# EUROPÄISCHE PATENTSCHRIFT

(11) **EP 4 051 089 B1**
(45) Veröffentlichungstag und Bekanntmachung des Hinweises auf die Patenterteilung: **17.06.2026**
(21) Anmeldenummer: 20800826.8
(22) Anmeldetag: 29.10.2020
(51) Int. Cl.: A61B 5/00, A61B 5/256, A61B 5/30, A61B 5/372, A61B 5/397, A61N 2/02, A61N 2/00

(54) **VORRICHTUNG ZUR BESTIMMUNG EINER SCHLAFAPNOE**
DEVICE FOR DETERMINING SLEEP APNOEA
DISPOSITIF DE DÉTERMINATION D'APNÉE DU SOMMEIL

(30) Priorität: 30.10.2019 DE 102019129288
(43) Veröffentlichungstag der Anmeldung: 07.09.2022
(73) Patentinhaber: Universitätsmedizin der Johannes Gutenberg-Universität Mainz, 55131 Mainz (DE)
(72) Erfinder: GOUVERIS, Haralampos, 55131 Mainz (DE); MUTHURAMAN, Muthuraman, 55234 Freimersheim (DE); SCHWABE, Matthias, 55131 Mainz (DE); ABRIANI, Ali, 24943 Flensburg (DE); BOEKSTEGERS, Philipp Tjarko, 55131 Mainz (DE); FASSNACHT, Sanja Mirjam, 55116 Mainz (DE); SCHMITT, Elena, 60316 Frankfurt/Main (DE); BAHR, Katharina, 55118 Mainz (DE)
(74) Vertreter: Patentanwälte Dr. Keller, Schwertfeger Partnerschaft mbB
(86) Internationale Anmeldenummer: PCT/EP2020/080393
(87) Internationale Veröffentlichungsnummer: WO 2021/084005

(56) Entgegenhaltungen:
- EP-A1- 2 767 235
- EP-A2- 0 199 214
- WO-A1-2017/201088
- WO-A2-2012/170816
- US-A1- 2017 087 360
- V K VARADAN ET AL: "Wearable Technology and Mobile Platform for Human Health Monitoring", FORUM FOR ELECTROMAGNETIC RESEARCH METHODS AND APPLICATION TECHNOLOGIES, vol. July-August, 1 July 2016 (2016-07-01), pages 1 - 38, XP055765875, Retrieved from the Internet <URL:https://www.e-fermat.org/files/articles/Varadan-ART-2016-Vol16-Jul_Aug-005%20Wearable%20Technology%20and%20Mobile%20Platform%20for.pdf> [retrieved on 20210118]

## Beschreibung

### Technisches Gebiet:

Die vorliegende Erfindung betrifft eine Vorrichtung zur Bestimmung einer Schlafapnoe, insbesondere eine Vorrichtung zur automatisierten Bestimmung einer Schlafapnoe. Ein Verfahren zur Bestimmung einer Schlafapnoe mit Hilfe einer solchen Vorrichtung ist Teil der Beschreibung, jedoch nicht Teil der Erfindung.

Die Erfindung wird durch die angefügten Patentansprüche definiert.

### Stand der Technik:

Eine Schlafapnoe ist eine nächtliche Atemstörung, bei der es im Schlaf immer wieder zu längeren Atemstillständen oder Teilatemstillständen kommt. Unter längeren Atemstillständen oder längeren Teilatemstillständen versteht man per Definition Atemstillstände oder Teilatemstillstände, die länger als 10 Sekunden dauern. Kurze, weniger als 10 Sekunden andauernde Atemaussetzer werden in der Regel als nicht gesundheitsschädlich angesehen. Sollte es während des Schlafs jedoch häufiger als fünf Mal pro Stunde Schlaf zu Atemaussetzern oder Teilatemaussetzern kommen, kann dies schwere gesundheitsschädliche Folgen haben.

Üblicherweise wird eine Schlafapnoe bzw. der Schweregrad einer Schlafapnoe in einem Schlaflabor mit Hilfe einer kardiorespiratorischen Polysomnographie festgestellt. Der Schweregrad wird hierbei aktuell in drei Schweregradgruppen eingeteilt.

Bei den bekannten Methoden werden die Atmung und respiratorische Ereignisse wie Hypopnoen (Teilatemaussetzer) oder Apnoen (komplette Atemaussetzer) im Schlaf mittels eines Atemflusssensors, zwei piezoelektrischen Dehngurten um den Brustkorb und um den Bauch sowie eines Pulsoximeters, das die arterielle Sauerstoffsättigung im Blut bestimmt, erfasst. Weiterhin werden die Gehirnströme mittels Elektroenzephalografie (EEG) und die Augenbewegungen mittels Elektrookulographie (EOG) aufgezeichnet. Schließlich wird auch der Muskeltonus im Kinnbereich überwacht, indem Klebelektroden am Kinn befestigt werden.

Bei der Messung des Atemflusses mit Hilfe eines Atemflusssensors handelt es sich um eine nasale Staudruckmessung über Nasenbrille und/oder eine Messung des oralen Atemstroms mittels eines oralen Thermistors.

Zur Bestimmung der Gehirnströme mittels Elektroenzephalografie ist eine Vielzahl von Elektroden notwendig, die am Kopf des Patienten angeordnet sind. Die Elektroenzephalografie dient dazu, eine Differenzierung zwischen Schlaf- und Wachphasen während der Nacht (bzw. während der Aufzeichnung), eine Schlafstadien-Klassifizierung während der Schlafphase und somit die Schlafarchitektur sowie respiratorische, motorische und physiologische Weckreaktionen, sogenannte Arousals, zu erfassen.

Die Vielzahl der eingesetzten Sensoren stört den Schlaf einer Testperson wesentlich, so dass sich die Frage stellt, ob die im Schlaflabor erzielten Messwerte repräsentativ für das Schlafen in häuslicher bzw. gewohnter Umgebung sind. Aus diesem Grund werden zum Teil Messungen an zwei aufeinanderfolgenden Nächten durchgeführt.

Aufgrund der hohen Anzahl erfasster Messdaten nimmt die Auswertung respiratorischer Ereignisse selbst bei automatischer Auswertung vergleichsweise viel Zeit in Anspruch. Da bisher bekannte automatisierte Auswertungen häufig nicht fehlerfrei arbeiten, ist in der Regel eine Kontrolle der Auswertung durch einen Experten notwendig. Diese Kontrolle, die im ungünstigen Fall eine vollständige visuelle Auswertung sein kann, ist sehr zeitaufwändig. Aktuell ist somit eine zuverlässige Klassifizierung der respiratorischen Ereignisse einer aufgefundenen Schlafapnoe nur mit Hilfe eines Experten möglich.

Aufgrund dessen, dass die zuverlässige Bestimmung von Atemaussetzern bzw. Teilatemaussetzern aufgrund einer Schlafapnoe aktuell noch sehr zeitaufwendig ist, werden Atemstillstände bzw. Teilatemaussetzer aufgrund einer Schlafapnoe häufig verspätet diagnostiziert, so dass den Menschen erst sehr spät therapeutisch geholfen wird.

Aus EP2767235 ist eine Vorrichtung zur Bestimmung von Schlafapnoe bekannt, die eine Kopfbedeckung mit EEG Elektroden an unter anderem den Positionen C3 und C4, sowie ein separates Kinnteil mit einer EMG Elektrode umfasst.

### Darstellung:

Es ist daher Aufgabe der vorliegenden Erfindung, eine Vorrichtung zur automatisierten Bestimmung von Schlafapnoezuständen bei einem Menschen bereitzustellen, welche den Schlaf eines Patienten weniger als bisher bekannte Vorrichtungen stört. Darüber hinaus ist ein Verfahren Teil der Beschreibung, jedoch nicht der Erfindung, mit dessen Hilfe es auf einfache Weise möglich ist, Schlafapnoezustände zuverlässig automatisch zu erkennen und welches darüber hinaus in der Lage ist, den Schweregrad einer aufgefundenen Schlafapnoe zu bestimmen.

Schließlich ist es Aufgabe der vorliegenden Erfindung, eine Vorrichtung zur Therapie von Atemstillständen bzw. Teilatemaussetzern im Zusammenhang mit einer Schlafapnoe bereitzustellen. Ein entsprechendes Verfahren ist Teil der Beschreibung, jedoch nicht Teil der Erfindung.

Der Erfindung liegt die Erkenntnis zugrunde, dass eine Kohärenz zwischen Elektroenzephalographiesignalen und Elektromyographiesignalen eine Aussage über Schlafapnoezustände, insbesondere über den Schweregrad einer Schlafapnoe ermöglicht.

Insbesondere basiert die Erfindung auf der Erkenntnis, dass die Kohärenz von EEG-Signalen an den Elektroenzephalographiestellen C3 und C4 und dem Muskeltonus am Kinn eine Klassifizierung des Schweregrads der Schlafapnoe beim Menschen ermöglicht.

Die erfindungsgemäße Vorrichtung zur Bestimmung des Schweregrads der Schlafapnoe greift somit auf die Elektroenzephalografie (EEG) und die Elektromyographie (EMG), mit deren Hilfe der Muskeltonus bestimmt werden kann, zurück.

Die Vorrichtung selbst umfasst eine Kopfbedeckung mit einem Kopfteil, das den Kopf eines Patienten zumindest an den Stellen bedeckt, an denen die Messstellen C3 und C4 der Elektroenzephalografie liegen, und mit einem Kinnteil, wobei das Kopfteil zwei Elektroden zur Erfassung von EEG-Signalen der Elektroenzephalographie an den Elektroenzephalographiestellen C3 und C4 und das Kinnteil wenigstens eine Elektrode zur Erfassung des EMG-Signals der Elektromyographie am Kinn aufweisen.

Dadurch, dass bei der vorliegenden Vorrichtung nur auf die Messsignale der Elektroenzephalographie und der Elektromyographie zurückgegriffen wird, kann die Vorrichtung zur Bestimmung der Schlafapnoe deutlich einfacher gestaltet werden.

Insbesondere kann auf die Atemflusssensoren verzichtet werden. Auch sind die bisher notwendigen piezoelektrischen Dehnungsgurte um den Brustkorb und um den Bauch sowie das Pulsoximeter, das die arterielle Sauerstoffsättigung im Blut bestimmt, nicht mehr notwendig. Es versteht sich, dass, wenn die Erfassung dieser Werte gewünscht ist, die vorliegende Vorrichtung zur Bestimmung einer Schlafapnoe dennoch mit diesen Messvorrichtungen kombiniert werden kann.

Bei den Elektroden zur Erfassung von EEG-Signalen und der wenigstens einen Elektrode zur Erfassung des EMG-Signals handelt es sich um Messelektroden. Die Anzahl der Messelektroden, die im Rahmen der Elektroenzephalographie notwendig sind, ist somit auf ein Minimum reduziert worden. In Summe sind nur drei notwendig. Gemäß der Erfindung, wie weiter unten angegeben ist, sind vier Messelektroden, nämlich zwei Elektroden zur Erfassung von EEG-Signalen der Elektroenzephalographie, an den Elektroenzephalographiestellen C3 und C4, und zwei Elektroden zur Erfassung des EMG-Signals der Elektromyographie am Kinn notwendig, um eine Messung zur Bestimmung einer Schlafapnoe sowie den Schweregrad einer Schlafapnoe durchzuführen.

Die Kopfbedeckung, bei der das Kopfteil und das Kinnteil einstückig miteinander verbunden sind, ist vergleichsweise einfach zu tragen, so dass sich die Schlafbedingungen während der Untersuchung kaum von denen unter normalen Umständen unterscheiden.

Um die EEG- Signale im Zusammenhang mit der Elektroenzephalographie zuverlässig korrekt zu erfassen, sind vorzugsweise eine Referenz- und eine Erdungselektrode vorgesehen. Vorzugsweise bedeckt die Kopfbedeckung auch die Stellen des Patienten, an denen die Referenz- und Erdungselektrode im Einsatz am Patienten vorgesehen sind.

Bei einer bevorzugten Ausführungsform ist wenigstens eine Elektrode fest in die Kopfbedeckung integriert, so dass sich die wenigstens eine Elektrode während der nächtlichen Untersuchung nicht versehentlich von der Kappe löst. Es versteht sich, dass sämtliche Elektroden fest in die Kopfbedeckung integriert sein können, um sicherzustellen, dass sich keine Elektrode während des Schlafens versehentlich löst.

Es hat sich herausgestellt, dass die Feststellung einer Schlafapnoe besonders gut möglich ist, wenn zwei Elektroden zur Erfassung des EMG-Signals vorgesehen sind, die jeweils den Muskeltonus am Kinn auf der rechten und linken Gesichtsseite eines Patienten erfassen. Aus diesem Grund ist es von Vorteil, dass die Kopfbedeckung der Vorrichtung gemäß der Erfindung eine erste Hälfte, eine zweite Hälfte sowie eine Längsachse aufweist, die die erste und zweite Hälfte voneinander trennt, wobei zwei Elektroden zur Erfassung des EMG-Signals vorgesehen sind, die jeweils auf einer Hälfte der Kopfbedeckung angeordnet sind. Bei einer bevorzugten Weiterbildung sind die zwei Elektroden symmetrisch zur Längsachse angeordnet, um zwei vergleichbare Messsignale zu erhalten.

Um den Schlaf eines Patienten möglichst wenig durch Messgeräte bzw. Messzubehör zu stören, ist es von Vorteil, dass die Elektrode zur Erfassung des EEG-Signals und/oder die Elektrode zur Erfassung des EMG-Signals eine schnurlose Elektrode ist.

Weiterhin hat es sich als vorteilhaft erwiesen, dass die Elektrode zur Erfassung des EMG-Signals eine Klebeelektrode ist. Das Vorsehen einer Klebeelektrode hat den Vorteil, dass die Elektrode besonders sicher an der gewünschten Messstelle am Kinn des Patienten während des Schlafens gehalten wird.

Bei einer bevorzugten Weiterbildung ist eine Datenverarbeitungsvorrichtung vorgesehen, die die EEG-Signale und das wenigstens ein EMG-Signal automatisch auswertet, um ohne großen Zeitaufwand respiratorische Ereignisse zu erkennen.

Hierbei ist es von weiterem Vorteil, dass zwischen der Datenverarbeitungsvorrichtung und den Elektroden eine schnurlose Kommunikation vorgesehen ist. Die Kommunikation zwischen den Elektroden, die die Messdaten erfassen, und der Datenverarbeitungsvorrichtung, die die erfassten Messdaten auswertet, ermöglicht eine einfache Auswertung der Messdaten. Bei regelmäßiger Datenübertragung während des nächtlichen Messvorgangs stehen die Daten somit bereits kurz nach Abschluss der Untersuchung am Morgen für eine Auswertung zur Verfügung. Eine kontinuierliche Übertragung der Messdaten bereits während der nächtlichen Untersuchung an eine Datenverarbeitungsvorrichtung ermöglicht sogar eine Online-Beobachtung des Patienten. Das Vorsehen einer schnurlosen Kommunikation vereinfach den Aufbau der Messvorrichtung, so dass der Schlaf des Patienten aufgrund der Übertragung der Daten an eine Datenübertragungseinheit nicht oder nur kaum beeinflusst wird.

Damit die Vorrichtung zur Bestimmung einer Schlafapnoe während des Schlafvorgangs nicht verrutscht, ist es von Vorteil, dass die Kopfbedeckung mützenartig ausgebildet ist und insbesondere den Hinterkopf im Wesentlichen vollständig bedeckt.

Um die Elektroden zur Bestimmung des Muskeltonus, insbesondere die Elektroden zur Bestimmung des EMG-Signals sicher an der gewünschten Messstelle zu halten, ist es von Vorteil, dass das Kinnteil als Kinnband ausgebildet ist.

Wie oben angeführt, ist es bevorzugt, dass zwei EMG-Messelektroden vorgesehen sind, die sich jeweils auf einer Hälfte der Kopfbedeckung befinden. Aus diesem Grund ist es zweckmäßig, dass ein erstes Kinnband an der ersten Hälfte der Kopfbedeckung und ein zweites Kinnband an der zweiten Hälfte der Kopfbedeckung vorgesehen sind.

Selbst wenn nur eine EMG-Messelektrode auf einer Gesichtshälfte verwendet wird, ist es von Vorteil, dass ein erstes Kinnband an der ersten Hälfte der Kopfbedeckung und ein zweites Kinnband an der zweiten Hälfte der Kopfbedeckung vorgesehen sind, da es dann in beiden Fällen möglich ist, die Kinnbänder stabil an dem Kinn des Patienten zu befestigen, indem das erste und zweite Kinnband miteinander verbindbar sind. Beispielsweise können die beiden Kinnbänder mittels eines Gurts verbunden werden oder einfach mit an den Kinnbändern angebrachten Schnüren nur verknotet werden. Bekannte alternative Verbindungsmöglichkeiten wie Klettverschlüsse sind ebenfalls einsetzbar.

Bei einer bevorzugten Weiterbildung der Vorrichtung zur Bestimmung einer Schlafapnoe sind Mittel zur Stimulation der Atmung vorgesehen. Somit dient die Vorrichtung zur Bestimmung der Schlafapnoe nicht nur der Bestimmung einer Schlafapnoe, insbesondere der Bestimmung des Schwergrads einer Schlafapnoe, sondern auch als Therapiegerät.

Als besonders effektives und kompaktes Therapiegerät hat sich eine Vorrichtung zur Bestimmung einer Schlafapnoe herausgestellt, bei der die Mittel zur Stimulation der Atmung wenigstens eine implantierbare Stimulationselektrode, eine Datenverarbeitungsvorrichtung zur Auswertung der EEG und/oder EMG-Signale, eine Steuereinheit und eine Vorrichtung zur Anregung der Stimulationselektroden aufweisen.

Um eine implantierte Stimulationselektrode anregen zu können, ist es von Vorteil, dass die implantierte Stimulationselektrode und die Vorrichtung zur Anregung der Stimulationselektrode jeweils eine magnetische Spule aufweisen, wobei vorzugsweise die Spule der Vorrichtung zur Anregung der Stimulationselektrode mit einer Batterie oder einem Akku verbunden sind. Wenn die Spule der Vorrichtung zur Anregung der Stimulationselektrode mit einer Batterie oder einem Akku verbunden ist, erfolgt die Stromversorgung der implantierten Stimulationselektrode von außen. Ein Aufladen des Akkus bzw. ein Batteriewechsel kann somit einfach durchgeführt werden.

Inhalt der vorliegenden Beschreibung ist auch ein Verfahren zur Bestimmung einer Schlafapnoe mittels einer erfindungsgemäßen Vorrichtung, umfassend die folgenden Schritte:
- Erfassen der elektrischen Aktivität des Gehirns mittels Elektroenzephalografie, wobei die mittels der durch Elektroenzephalografie erfassten EEG-Messsignale die Messsignale an den Elektroenzephalographiestellen C3 und C4 sind,
- Erfassen der elektrischen Muskelaktivität mittels Elektromyographie, wobei das mittels Elektromyographie erfasste EMG-Messsignal ein Messsignal im Bereich des Kinns ist,
- Korrelieren der erfassten EEG-Messsignale an den Stellen C3 und C4 und des erfassten EMG-Signals im Bereich des Kinns,
- Auswerten der korrelierten EEG-Messsignale an den Stellen C3 und C4 und des EMG-Signals im Bereich des Kinns in Bezug auf das Auftreten von Schlafapnoe-assoziierten respiratorischen Ereignissen.

Bei einer bevorzugten Weiterbildung erfolgt das Korrelieren der erfassten EEG-Messsignale an den Stellen C3 und C4 und des erfassten EMG-Signals im Bereich des Kinns und das Auswerten der korrelierten EEG-Messsignale an den Stellen C3 und C4 und des EMG-Signals im Bereich des Kinns in Bezug auf das Auftreten von Schlafapnoe-assoziierten respiratorischen Ereignissen automatisch.

Mit Hilfe der beschriebenen Verfahren lässt sich nicht nur das Vorliegen einer Schlafapnoe sondern auch der Schweregrad einer Schlafapnoe bestimmen.

Bei einer bevorzugten Weiterbildung des beschriebenen Verfahrens werden die EEG-Signale an den Stellen C3 und C4 und die EMG Signale im Bereich des Kinns unmittelbar nach der Erfassung dieser Signale korreliert und ausgewertet. Die ausgewerteten Daten werden unmittelbar nach der Auswertung an eine Steuereinheit übermittelt. Die Steuereinheit sendet in Abhängigkeit von den erhaltenen ausgewerteten Daten Steuersignale an eine Vorrichtung zur Anregung von implantierten Stimulationselektroden.

Dieses Verfahren, bei dem die Anregung von implantierten Stimulationselektroden noch während eines erfassten Atemaussetzers erfolgt, eignet sich nicht nur zur Bestimmung von Schlafapnoezuständen sondern auch zur Therapie von Patienten mit Schlafapnoe. Die Bestimmung von Schlafapnoezuständen sowie die Therapie von Patienten mit Schlafapnoe kann zeitgleich durchgeführt werden, was für den Patienten erhebliche Vorteile bringt.

Die Signalübertragung von der Vorrichtung zur Anregung von implantierten Stimulationselektroden zu den implantierten Stimulationselektroden kann per Funk, induktiv oder kapazitiv erfolgt. Bevorzugt ist die Verwendung von Anregespulen.

Es besteht die Möglichkeit bei jedem Atemzyklus die Stimulationselektroden anzuregen, so dass immer gewährleistet ist, dass der Patient keine Atemaussetzer hat. Es wurde jedoch festgestellt, dass dieses Verfahren den Nachteil hat, dass es sehr viel elektrische Energie benötigt, da die Stimulationselektroden im Wesentlichen kontinuierlich mit vergleichsweise viel Energie versorgt werden müssen. Bei einer bevorzugten Weiterbildung sendet die Steuereinheit nur dann Steuersignale an die Vorrichtung zur Anregung von implantierten Stimulationselektroden, wenn die von der Datenverarbeitungsvorrichtung erhaltenen Daten den Zustand einer Apnoe, sei es ein vollständiger Atemstillstand oder ein Teilatemaussetzer, darstellen. Somit kann der Energieverbrauch zur Therapie von Schlafapnoe deutlich reduziert werden.

### Kurze Beschreibung der Zeichnungen:

Bevorzugte Ausführungsformen der vorliegenden Erfindung werden anhand der beigefügten Zeichnungen erläutert, welche zeigen:
- Fig. 1: eine erste Ausführungsform einer Vorrichtung zur Bestimmung des Schweregrads einer Schlafapnoe, welche von einem Patienten getragen wird,
- Fig. 2: eine zweite Ausführungsform einer Vorrichtung zur Bestimmung des Schweregrads einer Schlafapnoe, welche von einem Patienten getragen wird und welche zusätzlich geeignet ist, Muskelfunktionen zu steuern,
- Fig. 3: einen Ausschnitt der in Fig. 2 dargestellten Ausführungsform und
- Fig. 4: einen Querschnitt durch eine Vorrichtung zur Anregung der Stimulationselektroden.

### Bester Weg zur Ausführung der Erfindung und gewerbliche Anwendbarkeit:

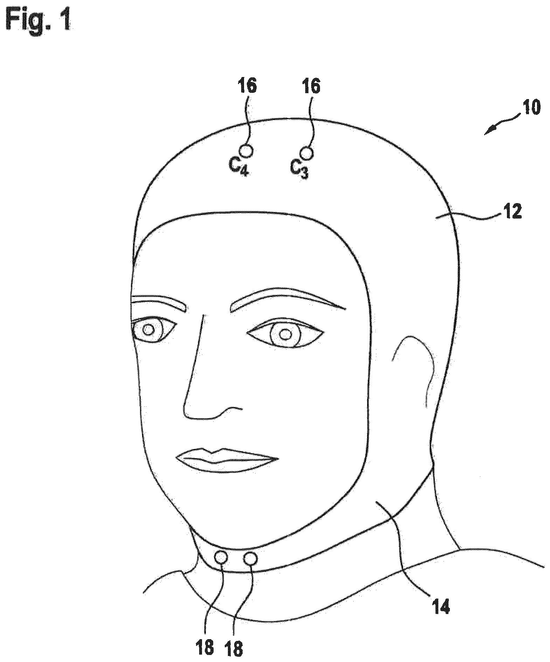

Fig. 1 zeigt eine Kopfbedeckung 10 einer ersten Ausführungsform einer Vorrichtung zur Bestimmung des Schweregrads einer Schlafapnoe mittels Elektroenzephalographie und Elektromyographie. Die Kopfbedeckung 10 beinhaltet nur vier Messstellen, an denen patientenbezogene Messdaten erfasst werden, wobei je zwei Messstellen zur Erfassung von patientenbezogenen Messdaten im Zusammenhang mit einer Elektroenzephalografie und die anderen beiden Messstellen zur Erfassung von patientenbezogenen Messdaten im Zusammenhang mit einer Elektromyographie dienen.

Patientenbezogenen Messdaten sind Messdaten, die zur Ermittlung von Schlafapnoezuständen herangezogen werden.

Die Kopfbedeckung umfasst ein Kopfteil 12 sowie ein Kinnteil 14. Kopfteil 12 und Kinnteil 14 sind miteinander verbunden, so dass die Kopfbedeckung einstückig ausgebildet ist.

Das Kopfteil 12 liegt eng am Kopf eines Patienten an und bedeckt den oberen Kopfbereich sowie den seitlichen Kopfbereich eines Patienten vollständig. Wenn auch nicht dargestellt, so kann sich das Kopfteil auch über den Hinterkopf des Patienten erstrecken.

Um einen angenehmen Tragekomfort zu erhalten, besteht die Kopfbedeckung aus einem Textilmaterial oder vergleichbarem flexiblen Material.

Auf dem Kopfteil 12 sind an den Positionen, die im angezogenen Zustand, in dem der Patient die Kopfbedeckung trägt, den Elektroenzephalographiestellen C3 und C4 entsprechen, Elektroden 16 zur Erfassung eines Elektroenzephalographiesignals (EEG-Signals) vorgesehen. Diese Elektroden dienen der Erfassung von patientenbezogenen Messdaten und sind somit Messelektroden.

Bei den Elektroden 16 zur Erfassung eines EEG-Signals handelt es sich um schnurlose Elektroden, die über eine Funkverbindung Daten an eine nicht dargestellte Datenverarbeitungsvorrichtung weitergeben. Die Elektroden 16 zur Erfassung eines EEG-Signals sind fest mit dem Kopfteil 12 verbunden.

Wenn auch nicht dargestellt so sind zusätzlich zu den Elektroden 16 zur Erfassung eines Elektroenzephalographiesignals (EEG-Signals), die als Messelektroden ausgelegt sind, eine Erdungselektrode und eine Referenzelektrode vorgesehen, die fest mit dem Kopfteil 12 verbunden sind. Die Erdungselektrode und die Referenzelektrode sind im Rahmen der Elektroenzephalographie sinnvoll, um korrekte, insbesondere absolute und nicht nur relative Messsignale der Messelektroden zu erhalten. Die Erdungselektrode und die Referenzelektrode dienen somit nicht der Erfassung von patientenbezogenen Messdaten sondern der Qualität der mittels der Elektroden 16 zur Erfassung eines EEG-Signals erfassten Signale.

Im Bereich des Kinns sind symmetrisch zur Gesichtsachse zwei Elektroden 18 zur Erfassung eines Elektromyographiesignals (EMG-Signals) vorgesehen. Die beiden Elektroden 18 zur Erfassung des EMG-Signals sind in dem Kinnteil 14 der Kopfbedeckung 10 integriert und somit fest mit der Kopfbedeckung 10 verbunden. Bei den beiden Elektroden 18 zur Erfassung des EMG-Signals handelt es sich um Klebeelektroden, die wie die Elektroden 16 zur Erfassung eines EEG-Signals schnurlos Daten an eine nicht dargestellte Datenverarbeitungsvorrichtung übertragen.

Das Kinnteil 14 ist als Kinnband ausgebildet und einstückig mit dem Kopfteil 12 verbunden, so dass die Kopfbedeckung 10 sturmhaubenartig ausgebildet ist.

Bei einer nicht dargestellten Ausführungsform kann das Kinnband zwei Kinnbänder aufweisen, die jeweils an einer Hälfte des Kopfteils 12 befestigt sind. Die beiden freien Enden des Kinnbandes können mittels eines Gurts, einer Schnalle oder einfach nur mittels Schnüren miteinander fest verbunden werden.

Zur Bestimmung des Schweregrads einer Schlafapnoe werden somit die EEG-Signale während des Schlafens an den C3- und C4-Stellen erfasst. Weiterhin werden während der nächtlichen Untersuchung die Signale des Muskeltonus am Kinn in Form von EMG-Signalen mittels der Elektromyographie aufgenommen. Die erfassten Messdaten werden in regelmäßigen Abständen per Funk an eine nicht dargestellte Datenverarbeitungsvorrichtung übermittelt. Dort werden sie in Bezug auf das Auftreten von respiratorischen Ereignissen ausgewertet. Dies kann online bereits während des nächtlichen Messvorgangs oder nach Beendigung des Schlafvorgangs offline erfolgen. Es ist sowohl eine optische Auswertung durch einen Experten als auch eine automatische Auswertung mittels eines Softwareprogramms möglich.

Die Auswertung erfolgt insbesondere durch die Korrelation der EEG-Signale an den C3- und C4-Stellen und den EMG-Signalen, die am Kinn des Patienten erfasst wurden.

Die EEG/EMG-Signalkombination bei gesunden Menschen unterscheidet sich deutlich von der EEG/EMG-Signalkombination, die bei Patienten erfasst wird, welche unter Hypopnoe oder Apnoe leiden. Mit Hilfe der EEG/EMG-Signalkombination ist somit eine Einteilung des Schweregrads einer Apnoe möglich.

Da bei dem beschriebenen Verfahren die Anzahl der erfassten Messwerte im Vergleich zu herkömmlichen Verfahren zu Einteilung des Schweregrads einer Apnoe deutlich reduziert ist, ist die Auswertung deutlich schneller und einfacher, ohne dass die Fehleranfälligkeit erhöht ist.

Bei der dargestellten Kopfbedeckung der Vorrichtung zur Bestimmung des Schweregrads einer Schlafapnoe wurde eine Kopfbedeckung gewählt, bei der das Kopfteil den Kopf im Wesentlichen vollständig bedeckt. Im Rahmen der Erfindung ist es jedoch ausreichend, wenn das Kopfteil der Kopfbedeckung die für die Messung des C3- und C4-Signals der Elektroenzephalographie notwendigen Stellen abdeckt. Es sollte jedoch sichergestellt sein, dass die Kopfbedeckung sich während der Untersuchung, d.h. während des Schlafvorgangs, nicht verschiebt.

Die beschriebene Vorrichtung zur Bestimmung des Schweregrads einer Schlafapnoe ist vergleichsweise einfach aufgebaut und somit kostengünstig herstellbar. Die feste Einbindung der Messelektroden in eine kappen- oder mützenartige Kopfbedeckung führt dazu, dass der Schlaf des Patienten während der nächtlichen Untersuchung kaum beeinflusst wird, wodurch Messergebnisse gewonnen werden, die mit hoher Wahrscheinlichkeit auch bei einem ungestörten Schlaf erfasst worden wären. Der einfache Aufbau der beschriebenen Vorrichtung ermöglicht es sogar, die Vorrichtung in häuslicher Umgebung einzusetzen, so dass die erfassten Messdaten die tatsächliche Schlafsituation zuhause bestmöglich wiedergeben.

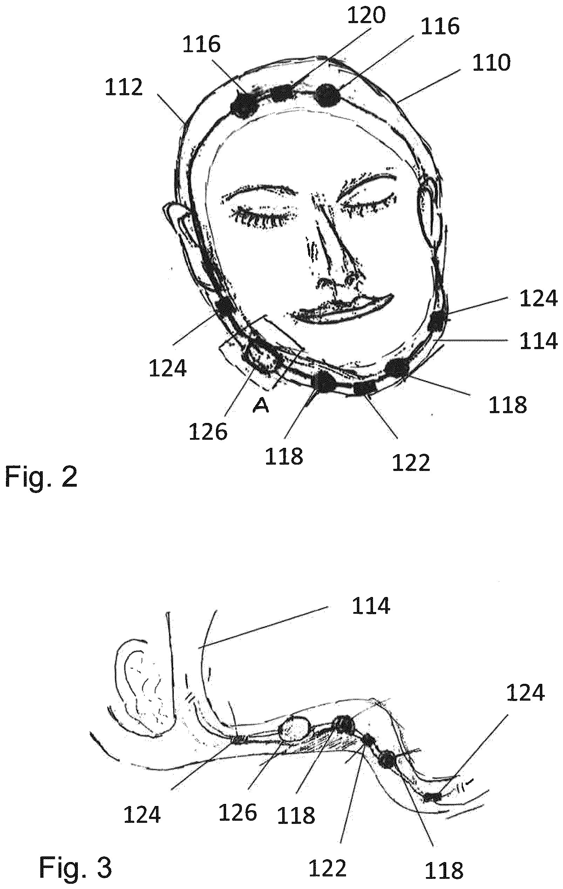

In den Figuren 2 bis 4 ist eine zweite Ausführungsform einer Vorrichtung zur Bestimmung des Schweregrads einer Schlafapnoe, welche von einem Patienten getragen wird, dargestellt. Mit Hilfe dieser zweiten Ausführungsform ist es möglich, zusätzlich Muskelfunktionen und/oder Nervenfunktionen zu steuern und somit Atemstillstände oder Teilatemaussetzer zu therapieren.

Wie die in Fig. 1 dargestellte Kopfbedeckung 10 umfasst die in den Fig. 2 bis 4 dargestellte Kopfbedeckung ein Kopfteil 112 sowie ein Kinnteil 114. Kopfteil 112 und Kinnteil 114 sind miteinander verbunden, so dass die Kopfbedeckung 110 einstückig ausgebildet ist. Weiterhin sind auf dem Kopfteil 112 an den Positionen, die im angezogenen Zustand, in dem der Patient die Kopfbedeckung trägt, den Elektroenzephalographiestellen C3 und C4 entsprechen, Elektroden 116 zur Erfassung eines Elektroenzephalographiesignals (EEG-Signals) vorgesehen. Im Bereich des Kinns sind symmetrisch zur Gesichtsachse zwei Elektroden 118 zur Erfassung eines Elektromyographiesignals (EMG-Signals) vorgesehen.

Zusätzlich zu der in Fig. 1 dargestellten Ausführungsform umfasst die Kopfbedeckung 110 einen ersten Verstärker 120, der der Verstärkung der Messsignale der Elektroenzephalographiesignale (EEG-Signals) dient und zwischen den beiden Elektroenzephalographiestellen C3 und C4 angebracht ist.

Zwischen den beiden Elektroden zur Erfassung des Elektromyographiesignals 118 ist ein zweiter Verstärker 122 vorgesehen.

Weiterhin ist auf jede Gesichtshälfte zwischen den jeweiligen Elektroden 116 zur Erfassung eines Elektroenzephalografiesignals (EEG-Signals) und Elektroden 118 zur Erfassung eines Elektromyographiesignals (EMG-Signals) ein Mikroprozessor 124 angeordnet. In dem Mikroprozessor 124 sind eine Datenverarbeitungsvorrichtung und eine Steuereinheit untergebracht.

Auf einer Gesichtshälfte ist unterhalb des Unterkieferrandes eine Vorrichtung zur Anregung einer Stimulationselektrode 126 vorgesehen.

Der erste und zweite Verstärker 120, 122, die Mikroprozessoren 124 und die Vorrichtung zur Anregung einer Stimulationselektrode 126 sind jeweils fest mit der Kopfbedeckung 110 verbunden.

Die Elektroden 116 zur Erfassung eines Elektroenzephalographiesignals (EEG-Signals), die Elektroden 118 zur Erfassung eines Elektromyographiesignals (EMG-Signals), der erste und zweite Verstärker 120, 122, die Mikroprozessoren 124 und die Vorrichtung zur Anregung einer Stimulationselektrode 126 sind über eine Kabelverbindung miteinander verbunden. Alternativ kann die Kommunikation der genannten Teile vollständig oder teilweise per Funk erfolgen.

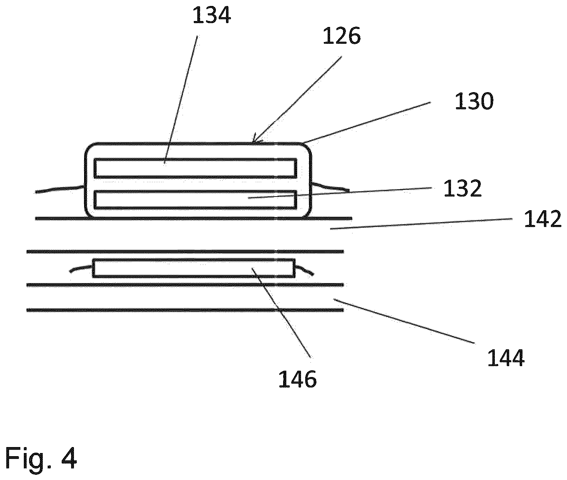

Wie in Fig. 4 dargestellt umfasst die Vorrichtung zur Anregung von Stimulationselektroden 126 eine in die Kopfbedeckung 110 integrierte Hülle 130, in der eine Spule 132 und eine Batterie 134 angeordnet sind.

Die magnetische Induktionsspule 146, die mit einer implantierten Stimulationselektrode in Verbindung steht, ist entweder zwischen Unterhaut 142 und Platysmamuskelschicht 144 (wie in Fig. 4) bzw. unterhalb der Platysmamuskelschicht 144 (nicht dargestellt) implantiert. Die implantierte Stimulationselektrode umhüllt den jeweiligen Nerv. Es handelt sich beispielsweise um eine sogenannte Cuff-Elektrode.

Die Batterie 134 bzw. der Akku ist austauschbar in der Hülle130 der Vorrichtung zur Anregung von implantierten Stimulationselektroden 126 angeordnet, so dass die Energieversorgung der implantierten Stimulationselektrode von außen erfolgen kann. Dies bietet für den Patienten erhebliche Vorteile.

Die genaue Position der Vorrichtung zur Anregung der Stimulationselektrode 126 ist somit so gewählt, dass diese auf der subkutan implantierten Spule 146, die mit der implantierten Stimulationselektrode eines Patienten in Verbindung steht, angeordnet ist.

Die beiden Elektroden zur Erfassung der EEG-Signale 116 sowie die beiden Elektroden zur Erfassung der EMG-Signale 118, die beiden Verstärker 120, 122, die beiden Mikroprozessoren 124 und die Vorrichtung zur Anregung von implantierten Stimulationselektroden 126 kommunizieren wie folgt:
Die von den beiden Elektroden zur Erfassung von EEG-Signalen 116 und den beiden Elektroden zur Erfassung von EMG-Signalen 118 erfassten Signale werden mittels des jeweiligen Verstärkers 120, 122 verstärkt an die Mikroprozessoren 124 übermittelt. In dem Mikroprozessor 124 werden die EEG-Signale und EMG-Signale in Hinblick auf das Vorliegen von Schlafapnoezuständen ausgewertet. Die in dem Mikroprozessor 124 enthaltene Steuereinheit sendet Signale an die Vorrichtung zur Anregung von implantierten Stimulationselektroden 126 zur Stimulation der Atmung, insbesondere zur Stimulation von Muskeln, die an der Atmung beteiligt sind. Mittels der Spule 132 in der Vorrichtung zur Anregung von implantierten Stimulationselektroden 126 und der Spule 146, die mit der Stimulationselektrode in Verbindung steht, wird ein Anregungssignal an den Muskel bzw. Nerv gegeben, um die Atmung zu stimulieren.

Vorzugsweise sendet die Steuereinheit nur dann Signale an die Vorrichtung zur Anregung von implantierten Stimulationselektroden 126, wenn die vom Mikroprozessor 124 erfassten Daten der EEG- bzw. EMG-Signale einen Atemaussetzer in Form einer Schlafapnoe bzw. Schlafhypopnoe erfasst haben. Alternativ kann die Vorrichtung zur Anregung von implantierten Stimulationselektroden 126 nur dann ein Signal an die implantierte Stimulationselektrode abgeben, wenn eine Schlafapnoe oder eine Hypopnoe vorliegt.

Beispielsweise können mittels eines adaptiven closed-loop Systems die Werte der EEG-EMG Kohärenz alle 5-10 Sekunden in Echtzeit bestimmt werden und diese Daten dann während der nächsten 5-10 Sekunden für die Stimulation der Atmung des Atemzyklus im Schlaf verwendet werden.

Wenn die Vorrichtung auch im Zusammenhang mit einer Stimulationselektrode im Mundbereich beschrieben wurde, so ist es möglich, die Vorrichtung zur Anregung von implantierten Stimulationselektroden auch an anderen Stellen zu positionieren. Die Vorrichtung zur Anregung von implantierten Stimulationselektroden wird vorzugsweise in der Kopfbedeckung so angeordnet, dass diese im vom Patienten getragenen Zustand an der implantierten Stimulationselektrode anliegt. Die implantierten Elektroden können sowohl Muskeln als auch Nerven stimulieren.

Die von dem Mikroprozessor erfassten und gesendeten Daten können gespeichert werden und einer späteren Auswertung, beispielsweise durch medizinisches Personal, zur Verfügung gestellt werden. Es versteht sich, dass die Auswertung der erfassten und gesendeten Daten auch online bzw. direkt nach der Messung erfolgen kann.

Wenn auch nicht dargestellt so können die Stimulationselektroden auch in einer anderen, im Stand der Technik bekannten Art und Weise angeregt werden.

Es versteht sich, dass die im Zusammenhang mit den Figuren beschriebenen

Ausführungsformen miteinander kombiniert werden können. Die Erfindung wird durch die angefügten Patentansprüche definiert.

## Patentansprüche

1. Vorrichtung zur Bestimmung einer Schlafapnoe mittels Elektroenzephalographie und Elektromyographie, umfassend eine Kopfbedeckung (10; 110) mit einem Kopfteil (12; 112), das den Kopf eines Patienten zumindest an den Stellen bedeckt, an denen die Messstellen C3 und C4 der Elektroenzephalographie liegen, und mit einem Kinnteil (14; 114), wobei das Kopfteil (12; 112) zwei Elektroden (16; 116) zur Erfassung von EEG-Signalen der Elektroenzephalographie an den Elektroenzephalographiestellen C3 und C4 und das Kinnteil (14; 114) wenigstens eine Elektrode (18; 118) zur Erfassung des EMG-Signals der Elektromyographie am Kinn aufweisen, wobei die Kopfbedeckung (10; 110) eine erste Hälfte, eine zweite Hälfte sowie eine Längsachse, die die erste und zweite Hälfte voneinander trennt, aufweist und wobei zwei Elektroden (18; 118) zur Erfassung des EMG Signals vorgesehen sind, die jeweils auf einer Hälfte der Kopfbedeckung (10; 110) angeordnet sind.

2. Vorrichtung nach Anspruch 1, **dadurch gekennzeichnet, dass** im Zusammenhang mit der Elektroenzephalographie eine Referenz- und eine Erdungselektrode vorgesehen sind.

3. Vorrichtung nach einem der vorhergehenden Ansprüche, **dadurch gekennzeichnet, dass** wenigstens eine Elektrode (16, 18) fest in die Kopfbedeckung integriert ist.

4. Vorrichtung nach einem der vorhergehenden Ansprüche, **dadurch gekennzeichnet, dass** die zwei Elektroden (18; 118) zur Erfassung des EMG-Signals symmetrisch zur Längsachse der Kopfbedeckung (10;110) angeordnet sind.

5. Vorrichtung nach einem der vorhergehenden Ansprüche, **dadurch gekennzeichnet, dass** die Elektrode (16; 116) zur Erfassung des EEG-Signals und/oder die Elektrode (18; 118) zur Erfassung des EMG-Signals eine schnurlose Elektrode ist.

6. Vorrichtung nach einem der vorhergehenden Ansprüche, **dadurch gekennzeichnet, dass** die Elektrode (18; 118) zur Erfassung des EMG-Signals eine Klebeelektrode ist.

7. Vorrichtung nach einem der vorhergehenden Ansprüche, **dadurch gekennzeichnet, dass** eine Datenverarbeitungsvorrichtung vorgesehen ist, die die EEG-Signale und das wenigstens eine EMG-Signal automatisch auswertet, wobei vorzugsweise zwischen der Datenverarbeitungsvorrichtung und den Elektroden (16, 18; 116, 118) eine schnurlose Kommunikation vorgesehen ist.

8. Vorrichtung nach einem der vorhergehenden Ansprüche, **dadurch gekennzeichnet, dass** die Kopfbedeckung (10; 110) mützenartig ausgebildet ist und insbesondere den Hinterkopf im Wesentlichen vollständig bedeckt.

9. Vorrichtung nach einem der vorhergehenden Ansprüche, **dadurch gekennzeichnet, dass** das Kinnteil (14; 114) als Kinnband ausgebildet ist.

10. Vorrichtung nach einem der vorhergehenden Ansprüche, **dadurch gekennzeichnet, dass** ein erstes Kinnband an der ersten Hälfte der Kopfbedeckung und ein zweites Kinnband an der zweiten Hälfte der Kopfbedeckung vorgesehen ist, wobei das erste und zweite Kinnband miteinander verbindbar sind.

11. Vorrichtung nach einem der vorhergehenden Ansprüche, **dadurch gekennzeichnet, dass** Mittel zur Stimulation der Atmung vorgesehen sind.

12. Vorrichtung nach Anspruch 11, **dadurch gekennzeichnet, dass** die Mittel zur Stimulation der Atmung wenigstens eine implantierbare Stimulationselektrode, eine Datenverarbeitungsvorrichtung zur Auswertung der EEG-Signale und/oder EMG-Signale, eine Steuereinheit und eine Vorrichtung zur Anregung der Stimulationselektroden (126) aufweisen.

13. Vorrichtung nach Anspruch 12, **dadurch gekennzeichnet, dass** die Stimulationselektrode und die Vorrichtung zur Anregung der Stimulationselektrode (128) jeweils eine magnetische Spule (132, 146) aufweisen.

## Claims

1. A device for determining sleep apnea by means of electroencephalography and electromyography comprising a headgear (10; 110) having a head part (12; 112) covering the head of a patient at least at the locations where the measuring points C3 and C4 of the electroencephalography are located, and having a chin part (14; 114), wherein the head part (12; 112) comprises two electrodes (16; 116) for detecting EEG signals of the electroencephalography at the electroencephalography points C3 and C4, and the chin part (14; 114) comprises at least one electrode (18; 118) for detecting the EMG signal of the electromyography on the chin, wherein the head gear (10; 110) has a first half, a second half, and a longitudinal axis separating the first and second halves, and wherein two electrodes (18; 118) for detecting the EMG signal are provided, each of which is arranged on one half of the headgear (10; 110).

2. The device according to claim 1, **characterized in that** a reference electrode and a ground electrode are provided in connection with the electroencephalography.

3. The device according to one of the preceding claims, **characterized in that** at least one electrode (16, 18) is securely integrated into the headgear.

4. The device according to one of the preceding claims, **characterized in that** the two electrodes (18; 118) for detecting the EMG signal are arranged symmetrically to the longitudinal axis of the headgear (10; 110).

5. The device according to one of the preceding claims, **characterized in that** the electrode (16; 116) for detecting the EEG signal and/or the electrode (18; 118) for detecting the EMG signal is a wireless electrode.

6. The device according to one of the preceding claims, **characterized in that** the electrode (18; 118) for detecting the EMG signal is an adhesive electrode.

7. The device according to one of the preceding claims, **characterized in that** a data processing device is provided which automatically evaluates the EEG signals and the at least one EMG signal, wherein preferably a wireless communication is provided between the data processing device and the electrodes (16, 18; 116, 118).

8. The device according to one of the preceding claims, **characterized in that** the headgear is designed as a cap and, in particular, covers the back of the head essentially completely.

9. The device according to one of the preceding claims, **characterized in that** the chin part (14; 114) is designed as chin strap.

10. The device according to one of the preceding claims, **characterized in that** a first chin strap is provided on the first half of the headgear and a second chin strap is provided on the second half of the headgear, wherein the first and the second chin straps are connectable to each other.

11. The device according to one of the preceding games, **characterized in that** means for stimulating respiration are provided.

12. The device according to claim 11, **characterized in that** the means for stimulating respiration comprise at least one implantable stimulation electrode, a data processing device for evaluating the EEG signals and/or EMG signals, a control unit, and a device for stimulating the stimulation electrodes (126).

13. The device according to claim 12, **characterized in that** the stimulation electrode and the device for stimulating the stimulation electrode (128) each have a magnetic coil (132, 146).

## Revendications

1. Dispositif de détermination d'une apnée du sommeil au moyen de l'électroencéphalographie et de l'électromyographie, comprenant un couvre-chef (10 ; 110) avec une partie de tête (12 ; 112), qui couvre la tête d'un patient au moins aux sites où se trouvent les sites de mesure C3 et C4 de l'électroencéphalographie, et avec une partie de menton (14 ; 114), la partie de tête (12 ; 112) comportant deux électrodes (16 ; 116) pour la détection de signaux EEG de l'électroencéphalographie aux sites d'électroencéphalographie C3 et C4, et la partie de menton (14 ; 114) comportant au moins une électrode (18 ; 118) pour la détection du signal EMG de l'électromyographie au menton, le couvre-chef (10 ; 110) présentant une première moitié, une seconde moitié ainsi qu'un axe longitudinal séparant la première et la seconde moitié, et deux électrodes (18 ; 118) pour la détection du signal EMG étant prévues, chacune étant disposée sur une moitié du couvre-chef (10 ; 110).

2. Dispositif selon la revendication 1, **caractérisé en ce qu'**une électrode de référence et une électrode de mise à la terre sont prévues en relation avec l'électroencéphalographie.

3. Dispositif selon l'une des revendications précédentes, **caractérisé en ce qu'**au moins une électrode (16, 18) est intégrée de façon fixe dans le couvre-chef.

4. Dispositif selon l'une des revendications précédentes, **caractérisé en ce que** les deux électrodes (18 ; 118) pour la détection du signal EMG sont disposées de manière symétrique par rapport à l'axe longitudinal du couvre-chef (10 ; 110).

5. Dispositif selon l'une des revendications précédentes, **caractérisé en ce que** l'électrode (16 ; 116) pour la détection du signal EEG et/ou l'électrode (18 ; 118) pour la détection du signal EMG est une électrode sans fil.

6. Dispositif selon l'une des revendications précédentes, **caractérisé en ce que** l'électrode (18 ; 118) pour la détection du signal EMG est une électrode adhésive.

7. Dispositif selon l'une des revendications précédentes, **caractérisé en ce qu'**un dispositif de traitement de données est prévu, qui évalue automatiquement les signaux EEG et au moins un signal EMG, une communication sans fil étant de préférence prévue entre le dispositif de traitement de données et les électrodes (16, 18 ; 116, 118).

8. Dispositif selon l'une des revendications précédentes, **caractérisé en ce que** le couvre-chef (10 ; 110) est en forme de bonnet et couvre en particulier l'arrière de la tête de manière essentiellement complète.

9. Dispositif selon l'une des revendications précédentes, **caractérisé en ce que** la partie de menton (14 ; 114) est réalisée sous forme de bandeau mentonnier.

10. Dispositif selon l'une des revendications précédentes, **caractérisé en ce qu'**un premier bandeau mentonnier est prévu sur la première moitié du couvre-chef et un second bandeau mentonnier sur la seconde moitié du couvre-chef, le premier et le second bandeau mentonnier étant raccordables l'un à l'autre.

11. Dispositif selon l'une des revendications précédentes, **caractérisé en ce que** des moyens de stimulation de la respiration sont prévus.

12. Dispositif selon la revendication 11, **caractérisé en ce que** les moyens de stimulation de la respiration comportent au moins une électrode de stimulation implantable, un dispositif de traitement de données pour l'évaluation des signaux EEG et/ou des signaux EMG, une unité de commande et un dispositif d'activation de l'électrode de stimulation (126).

13. Dispositif selon la revendication 12, **caractérisé en ce que** l'électrode de stimulation et le dispositif d'activation de l'électrode de stimulation (128) comportent chacun une bobine magnétique (132, 146).
